# EUROPEAN PATENT APPLICATION

(11) **EP 1 764 123 A1**
(43) Date of publication of application: **21.03.2007**
(21) Application number: 06018410.8
(22) Date of filing: 02.09.2006
(51) Int. Cl.: A61M 25/00, A61M 39/24

(54) **Medical device with a check valve and a branch tube**

(30) Priority: 15.09.2005 JP 2005268142
(71) Applicant: Enomoto Co., Ltd., Uenohara-city Yamanashi 409-0198 (JP)
(72) Inventor: Enomoto, Nobuo, c/o Enomoto Co., Ltd., Uenohara-city, Yamanshi 409-0198 (JP); Yamada, Kazuo, c/o Enomoto Co. Ltd., Uenohara-city, Yamanashi 409-0198 (JP); Hosoda, Mitsuji, c/o Enomoto Co. Ltd., Uenohara-city, Yamanashi 409-0198 (JP); Ohkanda, Kouki, c/o Enomoto Co. Ltd., Uenohara-city, Yamanashi 409-0198 (JP); Osada, Yoshimichi, c/o Enomoto Co. Ltd., Uenohara-city, Yamanashi 409-0198 (JP)
(74) Representative: Luderschmidt, Schüler & Partner

(57) **Abstract**

There is provided a medical device, especially an indwelling needle with a branch tube, in which the construction is simple, the operation can be performed easily in a short period of time, and misoperation is less liable to occur, thus a medical accident being less prone to take place. In the medical device, a first case portion (31) fitted with a check valve (4) therein and provided with a flexible portion (32) having a pressing button (33) and a second case portion (35) connecting with the first case portion on the inflow side of the check valve are separate parts. The medical device is characterized by having a branch tube (36) which extends to the side of the second case portion and the interior of which communicates with the hollow portion of the second case portion.

## Description

### BACKGROUND OF THE INVENTION

### Field of the Invention

The present invention relates to a medical device used for treatment of human beings and animals. The medical device includes an indwelling needle (outer needle) for catheter, an inner needle for catheter, a wing-shaped needle, a plug for catheter, a plug for wing-shaped needle, a drip tube, and a plug for drip tube.

### Description of the Related Art

The applicant has proposed an indwelling needle (outer needle) for catheter incorporating a check valve in Japanese Patent Laid-Open No. 2003-260132, has proposed an outer needle for catheter and a wing-shaped needle that incorporate a check valve in Japanese Patent Laid-Open No. 2004-000374, has proposed a plug for catheter incorporating a check valve in Japanese Patent Laid-Open No. 2004-041334, and has further proposed a medical device and an animal treating device, in which a check valve is arranged in a frame provided with a flexible portion, and a push of the flexible portion with a finger temporarily brings the check valve to an open state, in Japanese Patent Laid-Open No. 2004-097524.

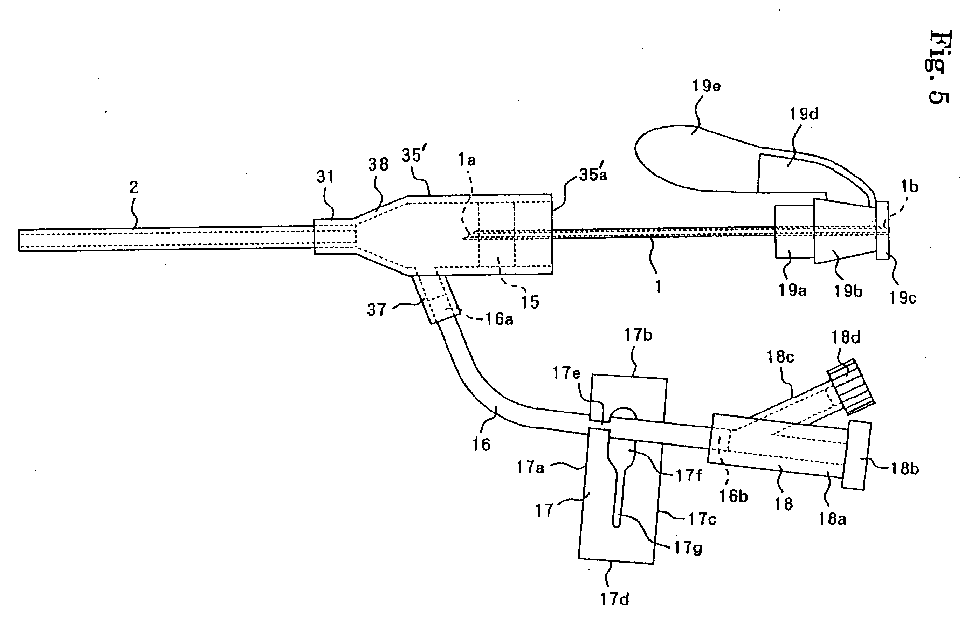

FIG. 5 is a schematic view of a conventional catheter with a branch tube.

A hollow cylindrical branch tube 37 projects from the side surface of a hollow cylindrical case portion 35' toward the slantwise rear. In an inner portion close to a rear end 35'a of the case portion 35', a rubber plate 15 is mounted, so that a liquid flow directed from the front of the case portion 35' toward the rear is stopped, and a metallic injection needle of an inner needle 1 for catheter can be inserted, penetrated, drawn, and closed from the rear. Between the rubber plate 15 and a front part 31 of the case portion 35' , a conical tapered portion 38 lies. To the front part 31 of the case portion 35', the rear end of a hollow cylindrical outer needle 2 for catheter, which is made of a soft resin, is connected and fixed. Also, to the branch tube 37, one end 16a of a tube 16 made of a soft resin or rubber is connected. The outer needle for catheter is also called an indwelling needle.

A tip end 1a of the inner needle 1 for catheter is cut slantwise so as to be sharp, and burrs etc. produced at the time of metalworking is removed so that a pain is less liable to be felt when the inner needle 1 is inserted in the skin. The rear portion of the inner needle 1 for catheter is joined to a part, in which an insertion fitting portion 19a, a central portion 19b, and a rear end portion 19c are arranged in this order, with the center axis being common. A rear end 1b of the inner needle 1 for catheter is arranged coincidently on the end surface of the rear end portion 19c. At the rear end 1b of the inner needle 1 for catheter, the hollow interior of the inner needle 1 for catheter is open, communicating with the atmosphere. On the side surface of the central portion 19b, a plate-shaped continuous portion 19d extending to one side and a gripping portion 19e for moving the inner needle 1 for catheter by being gripped by fingers are mounted sequentially in this order. These parts are made of a hard resin.

A plug 18 with a side branch tube has a hollow cylindrical side branch tube 18c projecting from the side surface of a hollow cylindrical portion 18a toward the slantwise rear. At the free end of the side branch tube 18c, a threaded cap 18d is mounted so as to be capable of being removed and closing. Also, to one end of the hollow cylindrical portion 18a, the other end 16b of the tube 16 is connected. The other end of the hollow cylindrical portion 18a is formed with a connecting flange 18b for threadedly engaging with a threaded cap, not shown.

In the center of the tube 16, an opening/closing plate 17 for opening and shutting off the tube is mounted. The opening/closing plate 17 has a rectangular plate shape, and a shutting-off narrow portion 17g and an opening wide portion 17f are cut out continuously from the opening/closing plate 17. Further, an attaching/detaching portion 17e for the opening/closing plate 17 to attach to and detach from the tube 16 is also cut out continuously. When the tube 16 is allowed to pass through the opening wide portion 17f, the tube 16 is not compressed, being brought to an open state, so that medicine or blood can flow. On the other hand, when the tube 16 is inserted in the shutting-off narrow portion 17g, the tube 16 is compressed, being brought to a shutoff state, so that medicine or blood cannot flow.

The construction of the conventional catheter with a branch tube shown in FIG. 5 is as described above. The function and use thereof is explained below.

The gripping portion 19e is gripped by fingers, and the outer needle 2 for catheter is pushed in precisely so that the tip end 1a of the inner needle 1 for catheter goes in the center of the rubber plate 15 in the case portion 35' , passing through the interior of the outer needle 2 for catheter, and slightly projects from the tip end of the outer needle 2 for catheter.

In the state in which the inner needle 1 for catheter is combined with the outer needle 2 in this manner, the tip end 1a of the inner needle 1 is inserted into the skin of a human being or animal and is allowed to arrive at a vein. Then, blood flows backward from the vein while passing through the interior of the inner needle 1 from the tip end 1a of the inner needle 1. However, since the inner needle 1 is made of stainless steel and is nontransparent, the blood in the inner needle 1 cannot be confirmed visually from the outside. At this time, the tube 16 is compressed by the shutting-off narrow portion 17g of the opening/closing plate 17 and thus is brought to a shutoff state.

After the outflow of blood from the rear end 1b of the inner needle 1 has been confirmed, the inner needle is pulled out. By pulling the tip end 1a of the inner needle 1 out of the rubber plate 15, the hole in the rubber plate 15 is eliminated, and becomes in a closing state. The tip end of the outer needle 2 is located in the vein.

Next, the tube 16 is sifted to the opening wide portion 17f of the opening/closing plate 17 to establish an open state. By doing this, the blood passes through the tube 16 due to the blood pressure of the vein, and flows into the plug 18 with a side branch tube. Since the side blanch tube 18c is closed up tight by the threaded cap 18d, the blood flows into the hollow cylindrical portion 18a. The air entrained in the indwelling needle with a branch tube is discharged through the connecting flange 18b together with the blood. Immediately after that, the tube 16 is shifted to the shutting-off narrow portion 17g of the opening/closing plate 17 to establish a shutoff state.

Successively, a threaded cap connected to one end of a drip tube is threadedly connected to the connecting flange 18b. The other end of the drip tube is attached to a medicine bag for drip.

Subsequently, the threaded cap 18d of the side branch tube 18c is loosened to discharge air bubbles entrained when the drip tube is connected. After the discharge of air bubbles has been finished, the threaded cap 18d is tightened to provide a seal.

Finally, the tube 16 is shifted to the opening wide portion 17f of the opening/closing plate 17 to establish an open state. Then, a drip is started.

When the drip is finished, the tube 16 is shifted to the shutting-off narrow portion 17g of the opening/closing plate 17 to establish a shutoff state, and thereafter the outer needle 2 for catheter is pulled out. Without the use of the opening/closing plate 17, a cock of the medicine bag for drip may be turned off to shut off the supply of medicine, and thereafter the outer needle 2 may be pulled out.

The conventional catheter with a branch tube shown in FIG. 5 has problems in that the number of parts are large, so that the product cost and the price are high; the operation is troublesome because many steps must be carried out, and thus misoperation is liable to occur; and the operation takes much time, so that the burdens on patients and medical staff are heavy.

### Summary of the Invention

The present invention has been made to solve the above problems, and accordingly an object thereof is to provide a medical device, especially an indwelling needle with a branch tube, in which the construction is simple, the operation can be performed easily in a short period of time, and misoperation is less liable to occur, thus a medical accident being less prone to take place.

The above obj ect is achieved by a medical device described in claim 1, namely, a medical device including as separate parts: a first case portion fitted with a check valve therein and provided with a flexible portion having a pressing button; and a second case portion connecting with the first case portion on the inflow side of the check valve, wherein the medical device has a branch tube which extends to the side of the second case portion and the interior of which communicates with the hollow portion of the second case portion.

In a preferred embodiment of the present invention, as described in claim 2, the medical device has a tube one end of which is fitted with a threaded hub and a threaded cap and the other end of which is connected to the branch tube.

Also, in another preferred embodiment of the present invention, as described in claim 3, threads are provided at the free end of the branch tube, and a threaded cap threadedly engaging with the threads is mounted.

Further, in still another preferred embodiment of the present invention, as described in claim 4, an inner needle is allowed to pass through a center axis and to pass through the center of the tip end of the check valve.

Still further, in still another preferred embodiment of the present invention, the first case portion and the second case portion are transparent or semitransparent.

### BRIEF DESCRIPTION OF THE DRAWINGS

FIG. 1 is a partial sectional view of a catheter in accordance with a first embodiment;
FIG. 2 is an assembly view of a catheter in accordance with a first embodiment;
FIG. 3 is an assembly view of a catheter in accordance with a second embodiment;
FIG. 4 is an assembly view of a catheter in accordance with a second embodiment;
FIG. 5 is a schematic view of a conventional catheter with a branch tube;
FIG. 6 is a schematic sectional view of a catheter in accordance with a third embodiment; and
FIG. 7 is an assembly view of a catheter in accordance with a third embodiment.

### DETAILED DESCRIPTION OF THE PREFERRED EMBODIMENTS

Embodiments of the present invention will now be described with reference to the accompanying drawings.

### First Embodiment

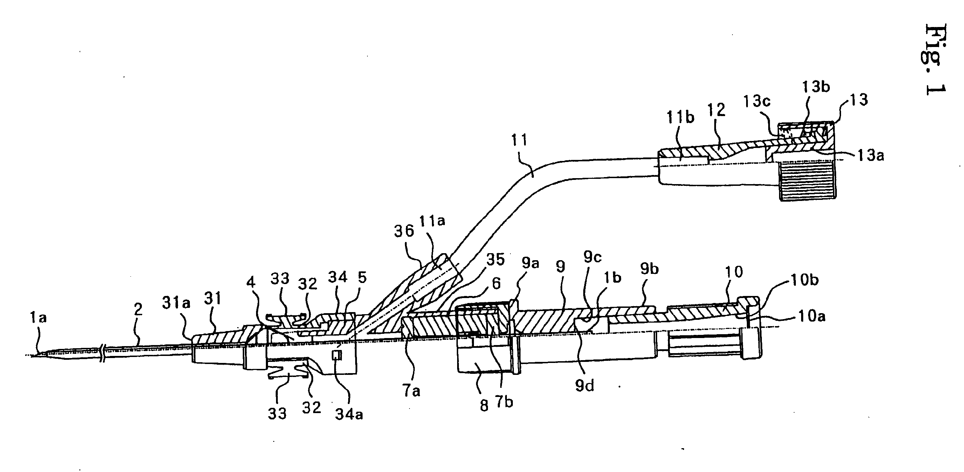

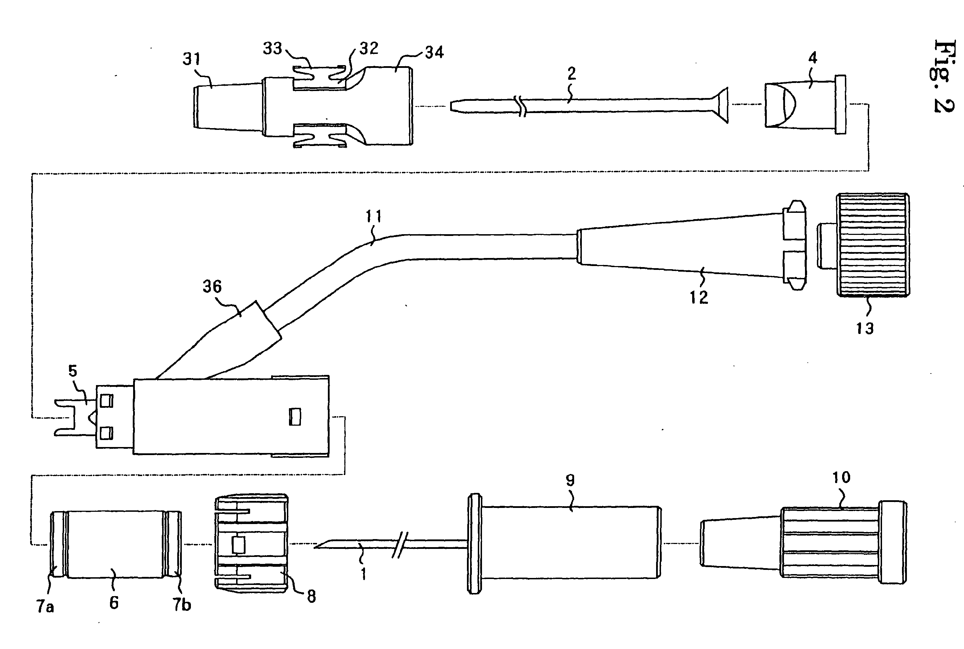

FIG. 1 is a partial sectional view of a catheter in accordance with a first embodiment, and FIG. 2 is an assembly view of the catheter in accordance with the first embodiment.

An outer needle for catheter in accordance with the first embodiment includes a first case portion, a second case portion 35, and an outer needle 2. In the outer needle for catheter, a check valve 4, a check valve holder 5, an inner needle guide 6, and sealing stoppers 7a and 7b are fitted, and in the rear part thereof, a cap 8 is fitted. The first case portion includes a front portion 31, a flexible portion 32, a pressing button 33 for pressing the flexible portion 32, and a joint portion 34. In the front portion 31, a part of the outer needle 2 made of a resin or rubber is inserted. The second case portion 35 has a hollow tubular shape. A branch tube 36 extends to the side slantwise with respect to the second case portion 35. The flexible portion 32 has a small thickness and is formed of a soft resin. Other portions of the first case portion are also formed of a soft resin, but has a large thickness. The first case portion and the second case portion 35 are transparent or semitransparent. In the middle part just under the flexible portion 32, a "drafting pen" portion of the check valve 4 is located. In the usual state, at the tip end of the "drafting pen" portion, the long sides of two flat plate portions are in contact with each other in a straight line form. On the inner surface in the center of the joint portion 34 of the first case portion, a notch portion 34a is formed to perform alignment in the circumferential direction of the check valve 4.

To the branch tube 36, one end 11a of a tube 11 is connected. The other end 11b of the tube 11 is connected to a threaded hub 12. In the vicinity of the free end of the threaded hub 12, a protrusion for threaded engagement is formed, and is threadedly engaged with a thread groove 13c formed on the inside of the outer surface of a screw 13. A nonslip groove is formed on the outside 13b of the screw 13. In the screw 13, an inner surface 13a is also formed.

For the inner needle for catheter in accordance with the first embodiment, the rear part of a stainless steel-made inner needle 1 is fixed to a hollow inner needle hub 9 so that the center axes thereof coincide with each other. A tip end 1a of the inner needle 1 is machined sharply. At the rear end of the inner needle hub 9, a plug 10 is insertedly mounted. An inner surface 10b of the plug 10 is isolated from the outside air by a shield film 10a. (The inner needle hub 9 and the plug 10 are preferably made of a transparent resin.)

When the tip end of the inner needle 1 for catheter is inserted from the rear end of the outer needle 2 for catheter, the inner needle 1 passes through the sealing stopper 7b, the inner needle guide 6, and the sealing stopper 7a in the named order while being guided, and advances along the center axis of the check valve 4. Then, the inner needle 1 passes through the center of the "drafting pen" portion, further passing through the interior of the outer needle 2, and is positioned so that the tip end 1a of the inner needle 1 projects slightly from the tip end of the outer needle 2.

### Second Embodiment

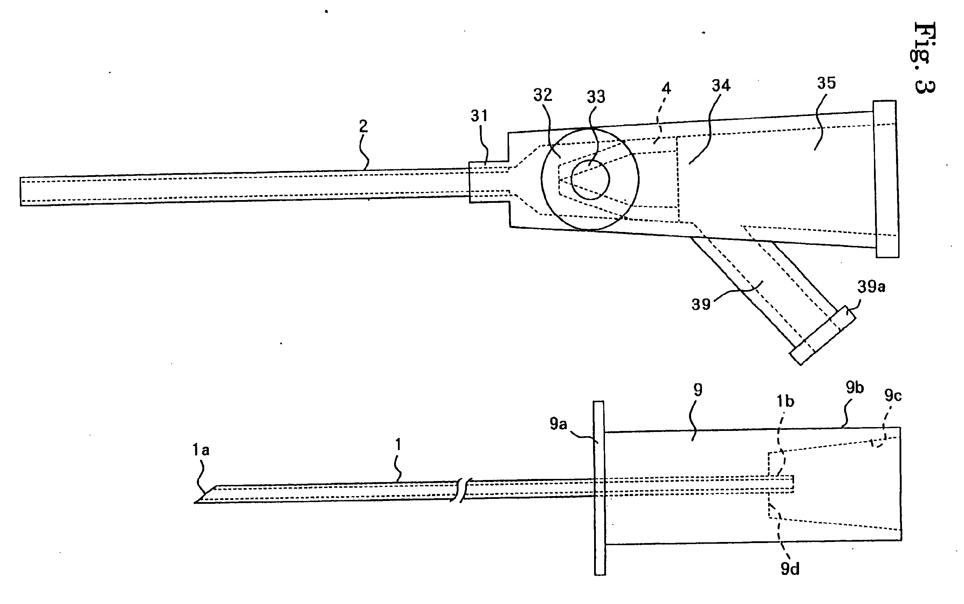

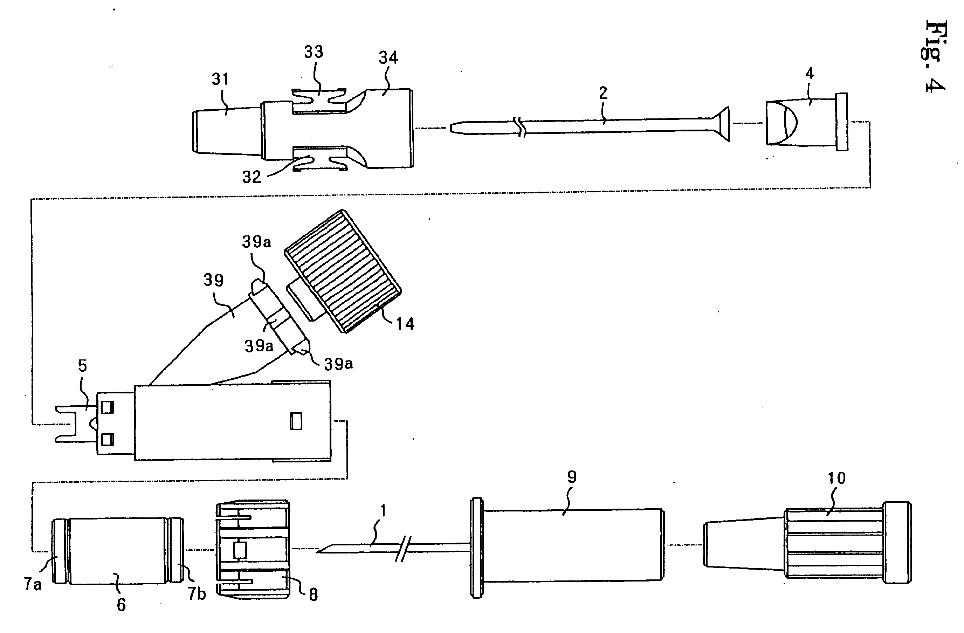

FIGS. 3 and 4 are assemblyviews of a catheter in accordance with a second embodiment.

The inner needle for catheter is almost the same as the inner needle for catheter of the first embodiment. A flange 9a is provided at the tip end of the inner needle hub 9 so that the inner needle for catheter can be inserted or drawn delicately by pushing or pulling the flange 9a with a finger of one hand. Also, in the rear part of the inner needle hub 9, there is formed a hollow portion having an inner side surface 9c and an inner bottom surface 9d. Also, the outer side surface 9b has a side surface shape of a column.

The outer needle for catheter is almost the same as the outer needle for catheter of the first embodiment.

However, a branch tube 39 differs from the branch tube 36 of the first embodiment in that no tube is connected thereto and the branch tube 39 consists of a threaded hub. The branch tube 39 extends to the side slantwise with respect to the second case portion 35. At the tip end of the branch tube 39, a protrusion 39a for threaded engagement for threadedly engaging with a screw, not shown, is formed.

The first case portion is formed with the flexible portion 32 like the first case portion of the first embodiment, and the pressing button 33 is attached to the flexible portion 32. The check valve 4 is arranged in the first case portion just under the flexible portion 32. Even if the check valve 4 is in a normal closed state, by pushing the pressing button 33, the check valve 4 is temporarily brought to an open state. If the pressing operation is stopped, the check valve 4 returns immediately to the closed state.

The above is an explanation of the catheters in accordance with two embodiments. In both of the embodiments, the backward flow of blood can be stopped by the check valve 4 merely by inserting the inner needle and the outer needle into the vein, and drawing the inner needle immediately after the backward flow of blood into the inner needle has been confirmed by visually observing the transparent inner needle hub 9. Moreover, the operation is to push or pull the flange 9a of the inner needle hub 9 with a finger or a nail, so that the operation can be performed easily and surely.

After the inner needle has been drawn, the sealing stoppers 7a and 7b become in a closed state, so that blood or medicine does not flow out automatically to the rear of the second case portion 35. In order to exhaust air entrained in the second case portion 35, the pressing button 33 is pushed to temporarily bring the check valve 4 to an open state, by which the air is released through the branch tube. After the finish of air release is confirmed visually, the pressing button 33 is released to bring the check valve 4 to a closed state.

Subsequently, the drip tube and the medicine bag are connected to the branch tube directly or indirectly. By a pressure produced by a difference in height between the medicine bag and the patient, the check valve 4 is opened, and thus the medicine etc. are injected into the vein in the normal direction.

### Third Embodiment

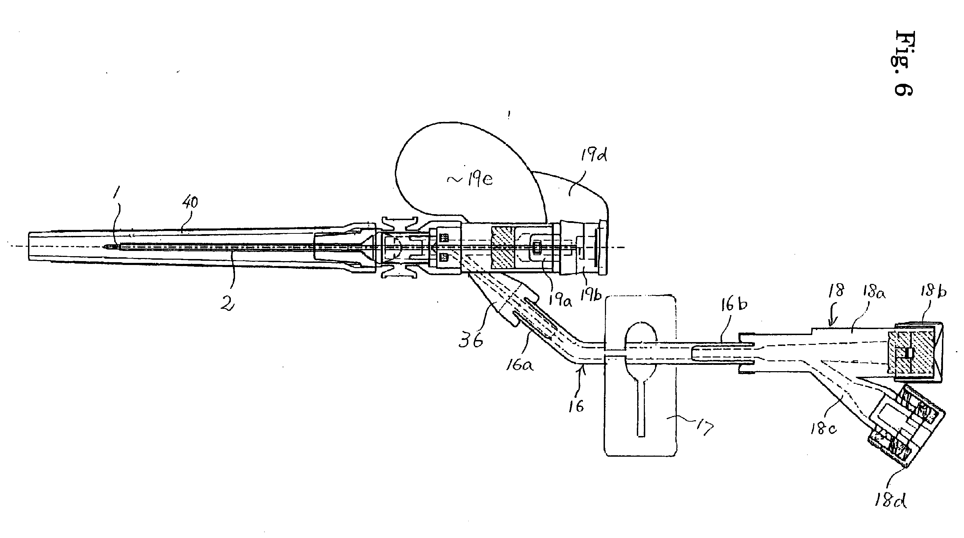

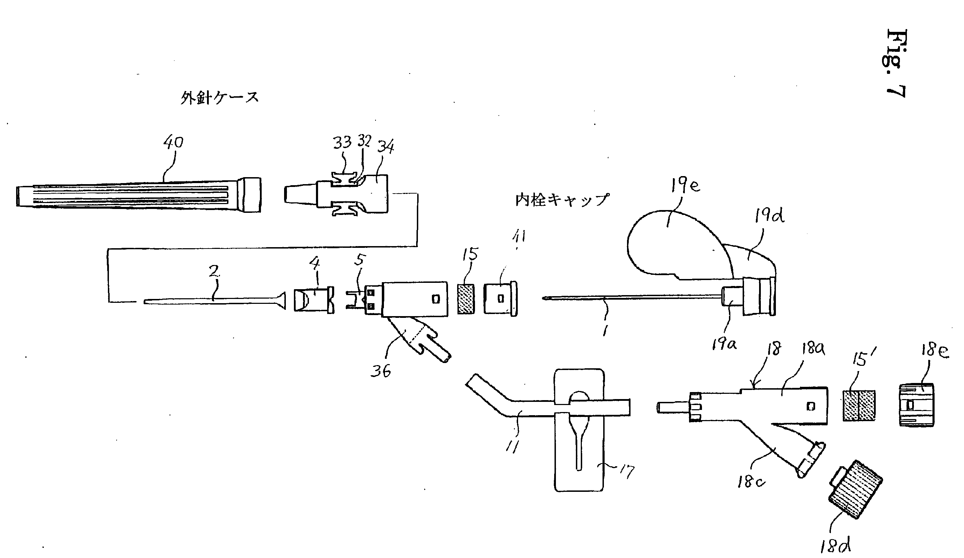

FIG. 6 is a schematic sectional view of a catheter in accordance with a third embodiment, and FIG. 7 is an assembly view of the catheter in accordance with the third embodiment.

Main differences of the third embodiment from the first and second embodiments are that on the side surface of a rear end portion 19c located in the rear part of the inner needle 1 for catheter, a plate-shaped continuous portion 19d extending to one side and a gripping portion 19e for moving the inner needle 1 for catheter by being gripped by fingers are mounted sequentially in this order, that an outer needle case 40 for covering the outer needle 2 for catheter is provided, and that in the center of a tube 16, an opening/closing plate 17 for opening and shutting off the tube is mounted.

## Claims

1. A medical device comprising as separate parts: a first case portion fitted with a check valve therein and provided with a flexible portion having a pressing button, and a second case portion connecting with the first case portion on the inflow side of the check valve, wherein
the medical device has a branch tube which extends to the side of the second case portion and the interior of which communicates with a hollow portion of the second case portion.

2. The medical device according to claim 1, wherein the medical device has a tube one end of which is fitted with a threaded hub and a threaded cap and the other end of which is connected to the branch tube.

3. The medical device according to claim 1, wherein threads are provided at a free end of the branch tube, and a threaded cap threadedly engaging with the threads is mounted.

4. The medical device according to any one of claims 1 to 3, wherein the medical device is used by causing an inner needle to pass through a center axis and to pass through a center of a tip end of the check valve.

5. The medical device according to any one of claims 1 to 4, wherein the first case portion and the second case portion are transparent or semitransparent.
